Europäisches Patentamt

European Patent Office

Office européen des brevets

(19)

(11) Publication number: **0 229 952**
**A2**

## (12) EUROPEAN PATENT APPLICATION

(21) Application number: **86116824.3**

(22) Date of filing: **03.12.86**

(51) Int. Cl.⁴: **C07C 1/20** , **C07C 1/24** ,
**C07C 11/02**

(30) Priority: **30.12.85 US 814426**

(43) Date of publication of application:
**29.07.87 Bulletin 87/31**

(84) Designated Contracting States:
**BE DE FR GB IT NL**

(71) Applicant: **MOBIL OIL CORPORATION**
**3225 Gallows Road**
**Fairfax Virginia 22037-0001(US)**

(72) Inventor: **Kaeding, Warren William**
**6 Roseberry Court**
**Lawrenceville New Jersey 08648(US)**

(74) Representative: **Kador & Partner**
**Corneliusstrasse 15**
**D-8000 München 5(DE)**

(54) A process for making light olefins from alcohols and ethers.

(57) A process for converting $C_1$-$C_4$ alcohols and/or $C_2$-$C_4$ to olefins over a catalyst comprising a zeolite in the presence of a $C_2$-$C_5$ straight chain alkane diluent. For example, methanol may be converted over ZSM-5 with a propane or n-butane diluent to produce an olefinic product rich in ethylene, propylene and butene, and containing a relatively small quantity of aromatics.

EP 0 229 952 A2

## A PROCESS FOR MAKING LIGHT OLEFINS FROM ALCOHOLS AND ETHERS

The present invention relates to the selective conversion of alcohols and ethers to olefins over a zeolite catalyst in the presence of an alkane diluent.

In order to provide an adequate supply of liquid hydrocarbons for use as synfuels or chemical feedstocks, various processes have been developed for converting coal and natural gas to gasoline, distillate and lubricants. A substantial body of technology has grown to provide oxygenated intermediates, especially methanol. Large scale plants can convert methanol or similar aliphatic oxygenates to liquid fuels, especially gasoline. However, the demand for heavier hydrocarbons has led to the development of processes for increasing yield of diesel fuel by a multi-stage technique.

Recent developments in zeolite catalysts and hydrocarbon conversion processes have created interest in utilizing olefinic feedstocks, for producing $C_5^+$ gasoline, diesel fuel, etc. U.S. Patents 3,960,978 and 4,021,502 disclose conversion of $C_2$-$C_5$ olefins, alone or with paraffinic components into higher hydrocarbons over crystalline zeolites having controlled acidity. Improved processing techniques are shown in U.S. Patents 4,150,062, 4,211,640 and 4,227,992.

Conversion of lower olefins, especially propene and butenes, over HZSM-5 is effective at moderately elevated temperatures and pressures. The conversion products are sought as liquid fuels, especially the $C_5^+$ aliphatic and aromatic hydrocarbons. Olefinic gasoline is produced in good yield and may be recovered as a product or recycled for further conversion to distillate-range products. Operating details are disclosed in U.S. Patents 4,445,031, 4,456,779 and 4,433,185.

In addition to their use as oligomerization catalysts, the ZSM-5 type catalysts are useful for converting methanol and other lower aliphatic alcohols or corresponding ethers to olefins. Particular interest has been directed to a catalytic process for converting low cost methanol to valuable hydrocarbons rich in ethene and $C_3^+$ alkenes. Various processes are described in U.S. Patents 3,894,107 (Butter et al), 3,928,483 (Chang et al), 4,025,571 (Lago), 4,423,274 (Daviduk et al) and 4,433,189 (Young). These processes can be optimized to produce a major fraction of $C_2$-$C_4$ olefins. Prior process proposals have included a separator to recover ethene rich vapor from water and $C_5^+$ hydrocarbon liquid phases. Oligomerization processes which favor the production of $C_{10}$-$C_{20}$ and higher aliphatics tend to convert only a small portion of ethene as compared to $C_3^+$ olefins.

The conversion of methanol to olefins over zeolites in the presence of diluents is described in Chang et al U.S. Patent Nos. 4,025,575 and 4,025,576 and in the Caesar et al U.S. Patent No. 4,083,888.

Despite the great advances which have been made, the conversion of methanol to olefins has not been as efficient as desired.

Accordingly, the present invention provides a process for converting a feed comprising at least one member of the group of $C_1$-$C_4$ alcohols and $C_2$-$C_4$ ethers to light olefins by contacting the feed with a catalyst comprising a zeolite having a constraint index of 1 to 12, characterized by adding to the feed a diluent of at least one straight chain $C_2$-$C_5$ alkane and obtaining at least a 15% conversion of the feed, on a diluent free basis, to a product comprising at least 70 wt % $C_2$-$C_4$ olefins and containing less than 10 wt % aromatics.

The catalysts used will contain at least one or more zeolites.

Zeolite, as used herein, means not only materials containing silicon and, optionally, aluminum atoms in the crystalline lattice structure thereof, but also materials which contain suitable replacement atoms for such silicon and/or aluminum. The term aluminosilicate zeolite means zeolites consisting essentially of silicon and, optionally, aluminum atoms in the crystalline lattice structure.

The zeolites useful herein have an effective pore size of 5 to 8 angstroms, such as to freely sorb normal hexane. In addition, the structure most provide constrained access to larger molecules. It is sometimes possible to judge from a known crystal structure whether such constrained access exists. For example, if the only pore windows in a crystal are formed by 8-membered rings of silicon and aluminum atoms, then access by molecules of larger cross-section than normal hexane is excluded and the zeolite is not of the desired type. Windows of 10-membered rings are preferred, although, in some instances, excessive puckering of the rings or pore blockage may render these zeolite ineffective.

Although 12-membered rings in theory would not offer sufficient constraint to produce advantageous conversions, it is noted that the puckered 12-ring structure of TMA offretite does show some constrained access. Other 12-ring structures may exist which may be operative for other reasons, and therefore, it is not the present intention to entirely judge the usefulness of the particular zeolite solely from theoretical structural considerations.

A convenient measure of the extent to which a zeolite admits molecules of varying sizes to its internal structure is the Constraint Index, which is determined is described fully in U.S. Patent No. 4,016,218. Constraint Index (CI) values for some typical materials are:

| | CI | (at test temperature) |
|---|---|---|
| ZSM-4 | 0.5 | (316°C) |
| ZSM-5 | 6-8.3 | (371°C - 316°C) |
| ZSM-11 | 5-8.7 | (371°C - 316°C) |
| ZSM-12 | 2.3 | (316°C) |
| ZSM-20 | 0.5 | (371°C) |
| ZSM-22 | 7.3 | (427°C) |
| ZSM-23 | 9.1 | (427°C) |
| ZSM-34 | 50 | (371°C) |
| ZSM-35 | 4.5 | (454°C) |
| ZSM-38 | 2 | (510°C) |
| ZSM-48 | 3.5 | (538°C) |
| ZSM-50 | 2.1 | (427°C) |
| TMA Offretite | 3.7 | (316°C) |
| TEA Mordenite | 0.4 | (316°C) |
| Clinoptilolite | 3.4 | (510°C) |
| Mordenite | 0.5 | (316°C) |
| REY | 0.4 | (316°C) |
| Amorphous Silica-alumina | 0.6 | (538°C) |
| Dealuminized Y | 0.5 | (510°C) |
| Erionite | 38 | (316°C) |
| Zeolite Beta | 0.6-2.0 | (316°C-399°C) |

Constraint Index seems to vary somewhat with severity of operations (conversion) and the presence or absence of binders. Likewise, other variables, such as crystal size of the zeolite, the presence of occluded contaminants, etc., may affect the C.I. It may be possible to so select test conditions, e.g. temperature, as to establish more than one value for the Constraint Index of a particular zeolite. This explains the range of Constraint Indices for some zeolites, such as ZSM-5, ZSM-11 and Beta.

Particular zeolites useful herein are exemplified by ZSM-5, ZSM-11, ZSM-12, ZSM-23, ZSM-35, ZSM-38, ZSM-48 and other similar materials.

ZSM-5 is described in U.S. Patents No. 3,702,886 and Re. 29,948.

ZSM-11 is described in U.S. Patent No. 3,709,979.

ZSM-12 is described in U.S. Patent No. 3,832,449.

ZSM-23 is described in U.S. Patent No. 4,076,842.

ZSM-35 is described in U.S. Patent No. 4,016,245.

ZSM-38 is described in U.S. Patent No. 4,046,859.

ZSM-48 is described in U.S. Patent No. 4,375,573.

It is crystal structure, as identified by the X-ray diffraction "fingerprint", which establishes the identity of a zeolite.

These zeolites, when prepared in the presence of organic cations, are substantially catalytically inactive, possibly because the intra-crystalline free space is occupied by organic cations from the forming solution. They may be activated by heating in an inert atmosphere at 540°C for one hour, for example, followed by base exchange with ammonium salts followed by calcination 540°C in air. The presence of organic cations in the forming solution may not be absolutely essential, but it helps. Generally, it is desirable to activate zaolites by base exchange with ammonium salts followed by calcination, e.g., in air at 540°C for 15 minutes to 24 hours.

Natural zeolites may sometimes be converted to zeolite structures of the class herein identified by various activation procedures and other treatments such as base exchange, steaming, alumina extraction and calcination, alone or in combinations. Natural minerals which may be so treated include ferrierite, brewsterite, stilbite, dachiardite, epistilbite, heulandite, and clinoptilolite.

Preferably the zeolites have a crystal framework density, in the dry hydrogen form, of not less than 1.6 grams per cubic centimeter.

In addition to the hydrogen form, other forms of the zeolite can be employed. Such other forms of the zeolite are those wherein the original alkali metal content has been reduced by 50 percent, usually to 0.5 percent by weight or less. Thus, the original alkali metal of the zeolite may be replaced by ion exchange with other suitable metal cations of Groups I through VIII of the Periodic Table, including, by way of example, nickel, copper, zinc, palladium, calcium or rare earth metals.

It is preferred to incorporate the zeolite with a matrix. Matrix materials include synthetic or naturally occurring substances as well as inorganic materials such as clay, silica and/or metal oxides. The latter may be either naturally occurring or in the form of gelatinous precipitates or gels including mixtures of silica and metal oxides.

Preferred matrix materials are alumina, silica-alumina, silica-magnesia, silica-zirconia, silica-thoria, silica-beryllia, and silica-titania, as well as ternary compositions, such as silica-alumina-thoria, silica-alumina-zirconia, silica-alumina-magnesia and silica-magnesia-zirconia. The matrix may be in the form of a cogel. The zeolite content may range from 1 to 99 wt %, usually 5 to 80 wt % of the dry composite.

An optional component of the conversion catalyst is a minor proportion, e.g., 0.05 to 50 wt % of the catalyst composite, of a difficultly reducible oxide, incorporated into the zeolite. This includes oxides of phosphorus or oxides of the metals of Groups IA, IIA, IIIA, IVA, VA, VIA, VIIA, VIIIA, IB, IIB, IIIB, IVB, or VB of the Periodic Chart of the Elements (Fisher Scientic Company, Catalog No. 5-702-10) which enhance the properties of the catalysts. The catalysts can be treated with phosphorus and/or magnesium compounds as in U.S. Patent Nos. 3,894,104; 4,049,573; 4,086,287; and 4,128,592.

Phosphorus, at least in part in the form of phosphorus oxide, can be added in an amount of 0.25 to 25 wt % of the catalyst composition, preferably 0.7 to 15 wt %. Such incorporation can be readily effected by contacting the zeolite composite with a solution of an appropriate phosphorus compound, drying and calcining. Preferred compounds are ammonium phosphates, including ammonium hydrogen phosphate, $(NH_4)_2HPO_4$, and ammonium dihydrogen phosphate, $NH_4H_2PO_4$.

Additional catalyst modifying procedures which may also optionally be employed to modify catalyst activity or selectivity include precoking and presteaming (e.g., before oxide incorporation), or combinations thereof.

## FEED

Alcohols and/or ethers are converted to light olefins, e.g., ethylene, propylene and butene. These alcohols may be $C_1-C_4$ alcohols, especially methanol, and these ethers may be $C_2-C_4$ ethers, e.g., dimethylether.

In addition to the alcohol or ether reactant, the feed also comprises at least one straight chain $C_2-C_5$ alkane diluent. Preferred alkane diluents are propane and n-butane. These alkane diluents may comprise from 5 to 95 wt % of the total weight of the alcohol and/or ether plus diluent. The molar ratio of diluent:alcohol or ether may be from 0.25:1 to 10:1. Other diluents, such as those described in the Chang et al U.S. Patent No. 4,025,575 and the Caesar et al U.S. Patent No. 4,083,888 may optionally be used in addition to the alkane diluent.

The $C_2-C_5$ straight chain alkane diluents may be a recycled stream. The n-alkane diluent promotes production of relatively high amounts of ethylene while minimizing formation of aromatics.

## REACTION CONDITIONS

Reaction conditions preferably include a temperature of 275°C to 600°C, a pressure of 0.5 to 50 atmospheres and a liquid hourly space velocity of 0.5 to 100.

Reaction conditions should be severe enough to convert at least 25 percent of the alcohol and ether feed, while producing a product containing more than 70 wt % $C_2$-$C_4$ olefins and less than 10 wt % aromatics, on a feed and diluent free basis. Preferably more than 50% of the feed is converted.

The light olefins of the present process may be recovered as products or further converted to gasoline and/or distillate. A process for further conversion is described in U.S. Patent No. 4,482,772. This further conversion may be tailored for the preferential formation of distillate, as noted in column 4, lines 12-30 of U.S. 4,482,772.

## CATALYST A -Mg-ZSM-5-POWDER

Ten grams of HZSM-5 crystal, prepared as in U.S. Patent No. 4,375,458, without binder, was converted to the ammonium form by treatment with three 100 ml, 1 molar solutions of ammonium nitrate. After washing with water, the catalyst was treated with 10 grams of $Mg(NO_3)_2.6H_2O$ dissolved in 10 grams of water, allowed to stand for 1 hour at room temperature, separated by filtration and dried in an oven at 110°C for 1 hr. The catalyst was heated in an oven at 550°C for 1 hour. Analysis indicated 5.1% Mg, crystallinity 93.3%, uncorrected for magnesium present as the oxide. Five grams of the catalyst powder was uniformly distributed on 37 cc of 14-20 mesh quartz. The alpha value was 28.

## CATALYST B -Mg-ZSM-5 -EXTRUDATE

The HZSM-5 crystals of Catalyst A were formed into an extrudate, 65 wt % zeolite with 35 wt % alumina binder.

The catalyst was impregnated with aqueous magnesium nitrate. Analysis indicated the presence of 6.9% magnesium present as the oxide. Pore volume is 0.492 cc/gm and an alpha value of 28 was determined.

## EXAMPLE 1

Methanol was converted over Catalyst A. (MgZSM-5, powder form). In a first series of runs, low temperatures were used to determine the threshold of reaction, and the effect of methanol/n-butane feed ratios. Large increases in methanol conversion occurred going from 275-300°C reminiscent of an autocatalytic effect. Several unexpected results appeared. Ethylene selectivities remained constant (34-36%) at 300°C and 325°C, Runs 3, 7, 8, 10, Table 1, when the molar methanol/n-butane feed ratios were changed from 1/3.1 to 1/0.6. This test, and all subsequent tests, were conducted at atmospheric pressure.

Table 1

n-Butane Diluent

| | Run 1 | Run 2 | Run 3 | Run 4 | Run 5 | Run 6 | Run 7 | Run 8 | Run 9 | Run 10 |
|---|---|---|---|---|---|---|---|---|---|---|
| Run No. | 1 | 2 | 3 | 4 | 5 | 6 | 7 | 8 | 9 | 10 |
| Temp., °C | 250 | 275 | 300 | 325 | 325 | 275 | 300 | 300 | 325 | 325 |
| WHSV – MeOH | 0.428 | .428 | .428 | .428 | .872 | .872 | .872 | .872 | 2.28 | 2.28 |
| WHSV – n-$C_4H_{10}$ | 2.40 | 2.40 | 2.40 | 2.40 | 2.39 | 2.39 | 2.39 | 2.39 | 2.39 | 2.39 |
| Mole Ratio of: $\frac{MeOH}{n\text{-}C_4H_{10}}$ | $\frac{1}{3.1}$ | $\frac{1}{3.1}$ | $\frac{1}{3.1}$ | $\frac{1}{3.1}$ | $\frac{1}{1.5}$ | $\frac{1}{1.5}$ | $\frac{1}{1.5}$ | $\frac{1}{1.5}$ | $\frac{1}{0.6}$ | $\frac{1}{0.6}$ |
| % Conversion: MeOH | 4 | 0 | 96 | 100 | 100 | 1 | 64 | 53 | 99 | 44 |
| n-$C_4H_{10}$ | –2 | 1 | –3 | –.4 | –.5 | –2 | –4 | –2 | –.2 | –3 |
| **Selectivity, wt %** | | | | | | | | | | |
| Methane | 0 | 4.1 | 1.6 | 1.5 | 1.6 | 0 | 1.0 | 1.0 | 1.1 | .7 |
| Ethylene | 0 | 25.1 | 36.5 | 21.4 | 12.2 | 0 | 36.4 | 36.9 | 31.4 | 34.4 |
| Ethane | 0 | 0 | 0 | .4 | .7 | 0 | 0 | 0 | .2 | 0 |
| Propylene | 0 | 42.8 | 15.4 | 12.3 | 13.9 | 0 | 29.2 | 34.0 | 14.6 | 33.2 |
| Propane | 100 | 28.0 | 8.1 | 15.9 | 22.9 | 16.3 | 4.6 | 4.1 | 8.7 | 2.7 |
| Butylene | 0 | 0 | 32.6 | 39.9 | 40.9 | 0 | 21.5 | 18.8 | 30.5 | 17.1 |
| Butane | SMa | SM | SM | SM | SM | SM | SM | SM | SM | SM |
| $C_5$-$C_{10}$ | 0 | 0 | 4.7 | 6.1 | 5.4 | 0 | 5.8 | 3.8 | 11.2 | 8.9 |
| Arom. | 0 | 0 | 1.1 | 2.5 | 2.4 | 83.7 | 1.5 | 1.4 | 2.3 | 3.0 |
| Total | 100.0 | 100.0 | 100.0 | 100.0 | 100.0 | 100.0 | 100.0 | 100.0 | 100.0 | 100.0 |

(a) SM = Starting Material

Furthermore, methanol conversion decreased in the same order. More important, butylene was a major product of reaction (30-40% selectivity). It is believed that butylene was produced from methanol, along with butane. Negative conversions for n-butane indicate a net production. Dimerization of ethylene is a probable route. Relatively small amounts of $C_5$+ paraffins and aromatics were produced.

In Table 2, additional tests were made with less n-butane diluent. Catalyst A was used.

Table 2

n-Butane Diluent

| Run No. | 11 | 12 | 13 | N₂-16[a] | 14 | 15 |
|---|---|---|---|---|---|---|
| Temp., °C | 325 | 325 | 325 | | 325 | 325 |
| WHSV MeOH | 1.49 | 1.49 | 1.49 | | 1.49 | 1.49 |
| n-$C_4H_{10}$ | 2.4 | 2.4 | 2.4 | | 1.47 | 1.47 |
| Moles $\frac{MeOH}{n\text{-butane}}$ | $\frac{1}{0.9}$ | $\frac{1}{0.9}$ | $\frac{1}{0.9}$ | | $\frac{1}{0.5}$ | $\frac{1}{0.5}$ |
| % Conversion: | | | | | | |
| MeOH | 68 | 93 | 94 | | 48 | 80 |
| n-$C_4H_{10}$ | − 2.9 | − 2.1 | − 1.6 | | − 4.9 | − 7.0 |

Selectivity, wt %

| | 11 | 12 | 13 | | 14 | 15 |
|---|---|---|---|---|---|---|
| Methane | .8 | .9 | .9 | | .8 | .7 |
| Ethylene | 35.8 | 32.9 | 32.9 | | 33.4 | 33.0 |
| Ethane | 0 | .1 | .1 | | .1 | .1 |
| Propylene | 31.7 | 23.4 | 22.6 | | 32.7 | 26.5 |
| Propane | 3.3 | 4.3 | 4.4 | | 2.5 | 3.4 |
| Butylene | 21.6 | 24.8 | 25.2 | | 18.2 | 21.8 |
| Butane | SM | SM | SM | | SM | SM |
| $C_5$-$C_{10}$ | 5.1 | 11.1 | 11.5 | | 8.9 | 11.5 |
| Arom. | 1.7 | 2.5 | 2.4 | | 3.4 | 3.0 |
| Total | 100.0 | 100.0 | 100.0 | | 100.0 | 100.0 |

(a) Flowing nitrogen, 16 hrs.

Substantial amounts of $C_2$-$C_4$ olefins (75-85%) were produced from methanol at 65-95% conversion. The reaction proceeded well even when small amounts of butane diluent were present as in Run 14 and Run 15.

EXAMPLE 2

Ten runs were made with Catalyst A, which had been calcined in air at 550°C for 16 hours, then reduced with $H_2$ for 1 hour. Methanol conversion went from 100 to 95%. After regeneration, 100% methanol conversion was restored. Average results for the 14 runs are:

| Selectivity | Ethylene | Propylene | Butylene | $C_2^=$ - $C_4^=$ |
|---|---|---|---|---|
| wt% | 31.8 | 16.4 | 31.3 | 79.5 |

Steady operation was observed for these runs. The negative conversion for the n-butane diluent indicates a net production of n-butane from methanol. The 80% selectivity to $C_2$-$C_4$ olefins is high.

In all tests, the methanol WHSV was constant at 0.87. The n-butance WHSV was constant at 2.4. This resulted in a mole ratio of MeOH to n-butane of 1:1.5.

## Table 3

### n-Butane Diluent

| | a | | | | | | b | | | c | | | | |
|---|---|---|---|---|---|---|---|---|---|---|---|---|---|---|
| Run No. | 1 | 2 | 3 | 4 | 5 | 6 | 7 | 8 | 9 | 10 | 11 | 12 | 13 | 14 |
| Temp., °C | 325 | 325 | 325 | 325 | 325 | 325 | 325 | 325 | 325 | 325 | 325 | 325 | 325 | 325 |
| Time, hrs. | 1 | 2 | 3 | 4 | 5 | 6 | 7 | 8 | 9 | 10 | 11 | 12 | 13 | 14 |
| **% Conversion:** | | | | | | | | | | | | | | |
| MeOH | 100 | 100 | 100 | 100 | 99 | 98 | 96 | 96 | 97 | 95 | 100 | 100 | 100 | 99 |
| n-$C_4H_9$ | − 2.3 | − 2.1 | − 1.8 | − 1.7 | − .8 | − .9 | − 1.9 | − 2.0 | − 1.7 | − 1.4 | − 2.7 | − 2.3 | − 2.3 | − 1.3 |

### Selectivity, wt %

| | | | | | | | | | | | | | | |
|---|---|---|---|---|---|---|---|---|---|---|---|---|---|---|
| Methane | 1.2 | 1.1 | 1.0 | 1.1 | 1.0 | 1.0 | 1.0 | 1.0 | 1.0 | 1.0 | 1.1 | 1.1 | 1.1 | 1.0 |
| Ethylene | 27.4 | 28.7 | 30.8 | 31.2 | 32.3 | 33.1 | 33.0 | 34.1 | 33.9 | 35.0 | 30.5 | 30.7 | 32.7 | 31.9 |
| Ethane | .3 | .2 | .2 | .2 | .2 | .2 | .1 | .1 | .1 | .1 | .2 | .2 | .2 | .2 |
| Propylene | 11.5 | 12.3 | 13.6 | 14.6 | 16.6 | 18.5 | 19.0 | 22.4 | 20.9 | 23.3 | 11.7 | 13.1 | 15.3 | 16.4 |
| Propane | 10.0 | 9.0 | 8.0 | 7.9 | 6.8 | 6.0 | 5.8 | 4.9 | 5.3 | 5.1 | 8.5 | 8.6 | 8.3 | 6.8 |
| Butylene | 34.8 | 34.4 | 33.2 | 33.6 | 30.9 | 29.3 | 29.3 | 26.8 | 28.1 | 27.7 | 33.8 | 32.3 | 33.2 | 30.6 |
| Butane | SM | SM | SM | SM | SM | SM | SM | SM | SM | SM | SM | SM | SM | SM |
| $C_5$-$C_{10}$ | 11.3 | 11.3 | 10.8 | 9.8 | 9.6 | 9.7 | 9.6 | 8.6 | 8.4 | 6.4 | 10.6 | 11.6 | 7.7 | 10.3 |
| Arom. | 3.5 | 3.0 | 2.4 | 1.6 | 2.6 | 2.2 | 2.2 | 2.1 | 2.3 | 1.4 | 3.6 | 2.4 | 1.5 | 2.8 |
| Total | 100.0 | 100.0 | 100.0 | 100.0 | 100.0 | 100.0 | 100.0 | 100.0 | 100.0 | 100.0 | 100.0 | 100.0 | 100.0 | 100.0 |

(a) Catalyst calcined with air, 16 hrs, 550°C, then treated with $H_2$ for 1 hr. before use.
(b) Catalyst flushed with nitrogen for 18 hrs.
(c) Catalyst calcined with air for 16 hrs, 550°C.

EXAMPLE 3

In this Example, the extrudate catalyst, Catalyst B, was tested. The methanol/n-butane molar feed ratio was 1/3. The WHSV of MeOH and 0.4, and that of n-butane was 2.4. Reaction conditions and results are summarized in Table 4.

Table 4

n-Butane Diluent

| Run No. | 1 | 2 | 3 | 4 | 5 | 6 |
|---|---|---|---|---|---|---|
| Temp., °C | 250 | 275 | 300 | 325 | 350 | 375 |
| **% Converson:** | | | | | | |
| MeOH | 14 | 1 | 1.5 | 96 | 100 | 100 |
| $n-C_4H_{10}$ | - 3 | - 2 | - 1 | - 2 | - .5 | - .5 |
| **Selectivity, wt %** | | | | | | |
| Methane | 0 | 0 | 9.6 | 1.9 | 2.1 | 2.0 |
| Ethylene | 0 | 0 | 33.8 | 31.5 | 19.2 | 10.9 |
| Ethane | 0 | 0 | 0 | 0 | .3 | .7 |
| Propylene | 0 | 0 | 0 | 22.1 | 16.6 | 19.2 |
| Propane | 13.0 | 13.0 | 5.7 | 5.6 | 13.3 | 20.6 |
| Butylene | 87.0 | 87.0 | 51.0 | 32.5 | 39.6 | 38.2 |
| Butane | SM | SM | SM | SM | SM | SM |
| $C_5-C_{10}$ | 0 | 0 | 0 | 5.0 | 6.9 | 6.5 |
| Arom. | 0 | 0 | 0 | 1.4 | 2.0 | 1.9 |
| Total | 100.0 | 100.0 | 100.0 | 100.0 | 100.0 | 100.0 |

In Table 5, the same catalyst was used, with the same WHSV of n-butane (2.4) but the methanol feed rate was increased to a 0.9 WHSV. The MeOH:n-butane mole ratio was 1:1.5.

Table 5

n-Butane Diluent

| Run No. | 7 | 8 | 9 | 10 | 11 |
|---|---|---|---|---|---|
| Temp., °C | 300 | 312 | 325 | 337 | 350 |
| **% Conversion:** | | | | | |
| MeOH | ~ 9 | ~ 9 | 51 | 77 | 98 |
| n-$C_4H_{10}$ | 2 | 2 | ~ 4 | ~ 3 | ~ 3 |

**Selectivity, wt %**

| | | | | | |
|---|---|---|---|---|---|
| Methane | 2.9 | 4.9 | 1.5 | 1.4 | 1.5 |
| Ethylene | 33.3 | 30.6 | 32.2 | 31.7 | 25.2 |
| Ethane | 0 | 0 | 0 | 0 | 0.2 |
| Propylene | 50.6 | 51.5 | 35.4 | 30.2 | 17.3 |
| Propane | 3.3 | 2.5 | 2.3 | 2.8 | 6.0 |
| Butylene | 0 | 0 | 22.9 | 24.5 | 35.3 |
| Butane | SM | SM | SM | SM | SM |
| $C_5$-$C_{10}$ | 7.2 | 7.6 | 4.4 | 7.4 | 11.3 |
| Arom. | 2.7 | 2.9 | 1.3 | 2.0 | 3.2 |
| Total | 100.0 | 100.0 | 100.0 | 100.0 | 100.0 |

Some scatter in selectivities is present at low conversions. However, performance at 50+% methanol conversion, 325-337°C, compares favorably to results with Catalyst A, which was a binderless powder.

| MeOH/n-$C_4H_{10}$ mol ratio | Conv. % | Temp. °C | CAT. | % Selectivity to Products | | | |
|---|---|---|---|---|---|---|---|
| | | | | ethylene | propylene | butylene | $C_2$-$C_4$ olefins |
| 1/3 | 96 | 325 | B | 31.5 | 22.1 | 32.5 | 86.1 |
| 1/1.5 | 51 | 325 | B | 32.2 | 35.4 | 22.9 | 90.5 |
| 1/1.5 | 77 | 337 | B | 31.7 | 30.2 | 24.5 | 86.4 |

EXAMPLE 4

Methanol was converted over Catalyst A using a propane diluent. Methanol was diluted with 3 moles of propane to determine the effect on products. The methanol WHSV was 0.428, the propane WHSV was 1.836. The mole ratio of propane:methanol was 3.1:1 Results are summarized in Table 6. Ethylene selectivities at 300°C were about 33%, $C_2$-$C_4$ olefins totalled 76% at 97% methanol conversion.

Table 6

Propane Diluent

| Run No. | 1 | 2 | 3 | 4 | 5 | 6 |
|---|---|---|---|---|---|---|
| Temp., °C | 275 | 287 | 300 | 300 | 325 | 350 |
| **% Conversion:** | | | | | | |
| MeOH | 1 | 8 | 97 | 97 | 100 | 100 |
| $n-C_3H_8$ | - .8 | - 1 | - 5 | - 2 | - 2 | - 2 |
| **Selectivity, wt %** | | | | | | |
| Methane | 0 | 1.7 | .8 | .8 | .9 | .9 |
| Ethylene | 9.0 | 28.4 | 33.2 | 32.6 | 21.8 | 13.1 |
| Ethane | 4.2 | 1.7 | .4 | .5 | .7 | .8 |
| Propylene | 16.6 | 32.8 | 16.7 | 16.4 | 12.1 | 14.3 |
| Propane | SM | SM | SM | SM | SM | SM |
| Butylene | 0 | 0 | 27.3 | 26.1 | 32.9 | 33.8 |
| Butane | 53.0 | 27.8 | 13.8 | 17.6 | 22.4 | 28.5 |
| $C_5-C_{10}$ | 0 | 5.7 | 5.6 | 4.6 | 5.7 | 5.2 |
| Arom. | 17.2 | 1.9 | 2.2 | 1.4 | 3.5 | 3.4 |
| Total | 100.0 | 100.0 | 100.0 | 100.0 | 100.0 | 100.0 |

Production of significant amounts of butane and butylene, in the absence of a butane diluent, strongly suggests that these products are produced from methanol. The negative propane conversion indicates a net production of propane also indicating that propane and propylene are produced from methanol. Results with propane are similar to tests with n-butane.


## EXAMPLE 5

To determine the effect of the magnesium modifier on the ZSM-5 catalyst, the original unmodified acid form of the identical batch was tested. The alpha value of the HZSM-5 was 200. The catalyst was calcined in air at 550°C, for 1 hour, before use. At constant methanol conversion, with n-butane diluent, the ethylene selectivities were reduced by roughly 50% in comparison to the Mg modified ZSM-5. More aromatics were made with the more active HZSM-5. Modification with magnesium reduced the alpha value from about 200 to 28 and inhibited aromatics formation.

In all tests, the WHSV of methanol was 0.43, while the WHSV of the n-butane was 2.4. The mole ratio of n-butane:methanol was 3:1.

Table 7

n-Butane Diluent

| Run No. | 1 | 2 | 3 | 4 |
|---|---|---|---|---|
| Temp., °C | 250 | 275 | 300 | 325 |
| % Conversion | | | | |
| $n\text{-}C_4H_{10}$ | – .7 | – 2.4 | – .9 | 6 |
| MeOH | 12 | 84 | 88 | 100 |
| **Selectivity, wt %** | | | | |
| Methane | 5.4 | 1.7 | 1.1 | .7 |
| Ethylene | 12.9 | 16.1 | 17.7 | 3.1 |
| Ethane | 0 | .3 | .6 | .6 |
| Propylene | 7.6 | 12.7 | 14.2 | 3.2 |
| Propane | 8.7 | 13.8 | 14.1 | 30.0 |
| Butylene | 27.2 | 18.6 | 16.6 | 15.9 |
| Butane | SM | SM | SM | SM |
| $C_5\text{-}C_{10}$ | 33.3 | 27.2 | 24.8 | 28.6 |
| Arom. | 4.9 | 9.6 | 10.9 | 17.9 |
| Total | 100.0 | 100.0 | 100.0 | 100.0 |

At the end of Run 4, the catalyst was calcined with air for 16 hours at 550°C.

## COMPARATIVE EXAMPLE A

Example 5 was repeated except that iso-butane was substituted for the n-butane diluent. The WHSV of iso-butane was 2.4, while the WHSV of the methanol was 0.43. The iso-$C_4$:methanol mole ratio was 3:1. Results are given in Table 8.

Table 8

Reaction of Methanol Diluted with iso-Butane
HZSM-5 (without binder)

C-2

| Run No. | 1 | 2 | 3 | 4 |
|---|---|---|---|---|
| Temp., °C | 250 | 275 | 300 | 325 |
| **% Conversion** | | | | |
| $n-C_4H_{10}$ | - .8 | - 3 | - 2 | 4 |
| MeOH | 11 | 97 | 94 | 100 |
| **Selectivity, wt %** | | | | |
| Methane | 9.0 | 1.6 | 1.0 | .7 |
| Ethylene | 11.2 | 10.2 | 16.7 | 3.1 |
| Ethane | 0 | .3 | .5 | .6 |
| Propylene | 8.2 | 4.5 | 13.2 | 3.2 |
| Propane | 0 | 12.4 | 13.4 | 24.2 |
| Butylene | 18.8 | 19.4 | 15.1 | 14.9 |
| Butane | SM | SM | SM | SM |
| $C_5-C_{10}$ | 46.3 | 43.7 | 24.2 | 32.6 |
| Arom. | 6.5 | 7.9 | 15.9 | 20.7 |
| Total | 100.0 | 100.0 | 100.0 | 100.0 |

Use of iso-butane diluent decreases selectivity for light olefins and increases production of heavier hydrocarbons and aromatics.

## COMPARATIVE EXAMPLE B

Cyclohexane (CH) was used as a diluent. Conditions of reaction and experimental results are summarized in Table 9. The catalyst was Catalyst A powdered Mg-ZSM-5. In the 250-325°C temperature range, CH was inert and 10-77% of the methanol was converted to hydrocarbons. Light olefins ($C_2-C_4$) ranged from 53-60% selectivity with relatively large amounts of ethylene (27-34%). At higher temperatures, higher aliphatics ($C_5-C_{10}$) and aromatics were produced and cyclohexane also reacted to contribute to products. Before use the Catalyst A was calcined with air at 550°C for 16 hrs.

Table 9

Cyclohexane Diluent

| Run No. | 1 | 2 | 3 | 4 | 5 | 6 | 7 | 8 |
|---|---|---|---|---|---|---|---|---|
| Temp., °C | 250 | 275 | 300 | 325 | 325 | 350 | 375 | 400 |
| **% Conversion** | | | | | | | | |
| MeOH | 10 | 24 | 54 | 77 | 100 | 100 | 100 | 100 |
| $C_y$-$C_6H_{12}$ | 1 | 1 | 1 | 1 | 2.7 | 7.6 | 12.5 | 77.5 |

**Selectivity, wt %**

| | | | | | | | | |
|---|---|---|---|---|---|---|---|---|
| Methane | 2.1 | 2.3 | 2.3 | 2.8 | 1.8 | 1.9 | 1.3 | 1.0 |
| Ethylene | 33.9 | 27.1 | 33.0 | 32.1 | 14.8 | 8.7 | 5.6 | 4.5 |
| Ethane | 0 | .1 | .3 | .4 | .5 | .7 | .8 | .7 |
| Propylene | 23.7 | 9.1 | 11.5 | 12.1 | 2.9 | 2.7 | 3.0 | 3.5 |
| Propane | 11.1 | 3.5 | 4.3 | 5.0 | 9.6 | 14.2 | 18.7 | 21.1 |
| Butylene | 0 | 8.5 | 8.4 | 9.0 | 6.5 | 6.2 | 5.8 | 6.0 |
| Butane | 0 | 11.6 | 9.2 | 5.5 | 14.2 | 14.6 | 15.7 | 15.4 |
| $C_5$-$C_{10}$ | 10.4 | 29.0 | 22.3 | 21.6 | 24.2 | 19.2 | 16.0 | 14.6 |
| Arom. | 18.8 | 8.8 | 8.7 | 11.5 | 25.5 | 31.8 | 33.1 | 33.2 |
| Total | 100.0 | 100.0 | 100.0 | 100.0 | 100.0 | 100.0 | 100.0 | 100.0 |

## COMPARATIVE EXAMPLE C

Methanol was diluted 50/50 by weight with water for these tests Catalyst A, which had been calcined in $N_2$ for 16 hours at 550°C, was used. Results are tabulated in Table 10. The major effect appears to be a reduction in activity. Only 16% methanol conversion was observed at 350°C, a temperature where 100% conversion was observed with all other diluents. Although about 36% ethylene and 82% $C_2$-$C_4$ olefins were produced, the methanol conversion was low. At higher temperatures with conversions of 77 and 100%, light olefin selectivities were severely reduced -23 to 12% ethylene and 65 to 50% for $C_2$-$C_4$ olefins.

### Table 10

### Water Diluent

| | C-16 | | | | | | |
|---|---|---|---|---|---|---|---|
| Run No. | | 69 | 70 | 71 | 72 | 73 | 74 |
| Temp., °C | | 300 | 325 | 350 | 375 | 400 | 425 |
| **% Conversion** | | | | | | | |
| MeOH | | 1 | 1 | 16 | 77 | 100 | 100 |
| **Selectivity, wt %** | | | | | | | |
| Methane | | | | .6 | .4 | .4 | .6 |
| Ethylene | | 55.0 | 46.0 | 35.8 | 23.1 | 12.4 | 15.3 |
| Ethane | | | | | .1 | .1 | .2 |
| Propylene | | 45.0 | 54.0 | 36.6 | 22.5 | 16.7 | 23.2 |
| Propane | | | | 2.0 | 2.9 | 2.3 | 2.7 |
| Butylene | | | | 9.2 | 19.8 | 21.4 | 27.7 |
| Butane | | | | 15.8 | 8.6 | 7.2 | 6.8 |
| $C_5-C_{10}$ | | | | | 11.9 | 21.6 | 8.3 |
| Arom. | | | | | 10.7 | 17.9 | 15.2 |
| Total | | 100.0 | 100.0 | 100.0 | 100.0 | 100.0 | 100.0 |

The WHSV for methanol was 2.06 and for water was also 2.06.
The $H_2O$:methanol mole ratio was 1.8:1.

### COMPARATIVE EXAMPLE D

Pure methanol was used in this series for conversion to hydrocarbons. The WHSV was 3.6. Catalyst A, which had been calcined 16 hours in $N_2$ at 550°C, was used. Results are summarized in Table 11. At 250-300°C, no hydrocarbons were found in the products. A temperature of 375°C was required to reach 100% methanol conversion. At relatively low conversion (14 and 41%), ethylene selectivity was 33 and 32% and $C_2$-$C_4$ olefins were 90 and 80%. At conversions of 80-100%, the latter was reduced from 90-80% to 65-50%.

## Table 11

### No Diluent

| Run No. | 20 21 22 | 23 | 24 | 83 | 25 | 84 | 26 | 27 |
|---|---|---|---|---|---|---|---|---|
| Temp., °C | 250 275 300 | 325 | 350 | 350 | 375 | 375 | 400 | 425 |
| WHSV MeOH | 3.6 | | | | | | | |
| % Conversion of MeOH | 0 | 14 | 41 | 80 | 97 | 95 | 100 | 100 |

Selectivity, wt %

| | | | | | | | | |
|---|---|---|---|---|---|---|---|---|
| Methane | 0 | .7 | .6 | .5 | .5 | .5 | .6 | .7 |
| Ethylene | 0 | 33.3 | 32.0 | 25.5 | 21.3 | 21.0 | 16.8 | 13.7 |
| Ethane | | | .1 | .2 | .2 | .2 | .2 | .2 |
| Propylene | | 34.8 | 28.8 | 16.5 | 15.1 | 14.8 | 11.2 | 12.5 |
| Propane | | 2.2 | 3.4 | 5.6 | 4.3 | 4.3 | 4.4 | 3.7 |
| Butylene | | 21.9 | 19.4 | 21.2 | 19.0 | 19.0 | 22.9 | 24.1 |
| Butane | | 7.0 | 5.7 | 9.0 | 8.1 | 8.2 | 9.4 | 9.3 |
| $C_5-C_{10}$ | | | 4.2 | 11.5 | 18.3 | 18.6 | 20.0 | 21.3 |
| Arom. | | .1 | 5.8 | 10.0 | 13.2 | 13.4 | 14.5 | 14.5 |
| Total | | 100.0 | 100.0 | 100.0 | 100.0 | 100.0 | 100.0 | 100.0 |

## Claims

1. A process for converting a feed comprising at least one member of the group of $C_1-C_4$ alcohols and $C_2-C_4$ ethers to light olefins by contacting the feed with a catalyst comprising a zeolite having a constraint index of 1 to 12, characterized by adding to the feed a diluent of at least one straight chain $C_2-C_5$ alkane and obtaining at least a 25% conversion of the feed, on a diluent free basis, to a product comprising at least 70 wt % $C_2-C_4$ olefins and containing less than 10 wt % aromatics.

2. The process of claim 1 further characterized in that the diluent is propane and/or n-butane.

3. The process of claim 1 further characterized in that conversion of feed occurs at a temperature of 275°C to 600°C, a pressure of 0.5 to 50 atmospheres and a liquid hourly space velocity of 0.5 to 100.

4. The process of any preceeding claim further characterized in that the feed is methanol.

5. The process of any preceeding claim further characterized in that the diluent is n-butane.

6. The process of any of claims 1 to 4 further characterized in that the diluent is propane.

7. The process of any preceeding claim further characterized in that the diluent is a recycle stream.

8. The process of any preceeding claim further characterized by at least 50% conversion of feed.